# EUROPEAN PATENT SPECIFICATION

(11) **EP 3 854 422 B1**
(45) Date of publication and mention of the grant of the patent: **22.01.2025**
(21) Application number: 19862426.4
(22) Date of filing: 06.09.2019
(51) Int. Cl.: A61L 15/20, A61K 9/70, A61K 47/18, A61K 47/32, A61K 47/44, A61L 15/24, A61L 15/34, A61L 15/42, A61L 15/44, A61P 25/04, A61K 9/00, A61L 26/00

(54) **BIOMATERIAL**
BIOMATERIAL
BIOMATÉRIAU

(30) Priority: 20.09.2018 JP 2018176358
(43) Date of publication of application: 28.07.2021
(73) Proprietor: FUJIFILM Corporation, Tokyo 106-8620 (JP)
(72) Inventor: CHIBA Kosuke, kami-gun, Kanagawa 258-8577 (JP); TAKAKU Koji, kami-gun, Kanagawa 258-8577 (JP)
(74) Representative: Klunker IP Patentanwälte PartG mbB
(86) International application number: PCT/JP2019/035212
(87) International publication number: WO 2020/059543

(56) References cited:
- WO-A1-95/34287
- JP-A- 2004 501 069
- JP-A- H02 237 917
- US-A1- 2013 177 606
- US-A1- 2014 186 279
- LEE, J. ET AL.: "In vitro peptide release from liquid crystalline buccal deli very systems", INT. J. PHARM., vol. 195, no. 1-2, 15 February 2000 (2000-02-15), pages 29 - 33, XP055693580

## Description

### BACKGROUND OF THE INVENTION

### 1. Field of the Invention

The present invention relates to a biomaterial.

### 2. Description of the Related Art

In cancer patients, cancer treatment affects the mucous membrane of the mouth and, stomatitis occurs easily. For example, in anti-cancer drug treatment, in radiation therapy for head and neck cancer (cancer in the range from the head to the neck) in a case where a drug that easily causes stomatitis is administered, when radiation is directly applied to the mucous membrane of the mouth, stomatitis is inevitable. The pain of stomatitis is so strong that it is difficult to eat a meal by mouth.

As a symptomatic treatment for stomatitis, a patch (for example, Aphthaseal^{(R)} 25 µg, manufactured by Taisho Pharmaceutical Co., Ltd., active ingredient: triamcinolone acetonide) that is directly attached to the affected part, an ointment (for example, Dexaltin oral ointment for oral cavity, manufactured by Nippon Kayaku Co., Ltd., active ingredient: dexamethasone) that is applied to the affected part, and a spray agent (for example, Salcoat^{(R)} capsule for oral spray 50µg, TEIJIN PHARMA LIMITED, active ingredient: beclomethasone propionate ester) that is sprayed on the affected part are mentioned.

When eating a meal by mouth, the patch attached to the affected part may be peeled off, or the ointment or the spray agent applied to the affected part may be lost, and thus the pain of stomatitis cannot be suppressed.

Accordingly, a biomaterial capable of suppressing such pain of stomatitis has been desired.

Particularly, for example, JP2008-169219A discloses a cosmetological composition for use for the aged and/or stressed skin, and in such a cosmetological composition in which water and at least one substance forming a lamellar structure together with the water are present in the above composition, the followings are further contained;
a) at least one compound having at least one functional group represented by General Formula I, and/or

   -CH₂-N⁺-(CH₃)₃ (Formula I)
b) at least one metabolite of this compound, and/or
c) S-adenosyl methionine.

Further, JP2008-509120A discloses a composition containing;
a) at least one monoacyl lipid,
b) at least one diacyl glycerol, at least one tocopherol, or a mixture thereof,
c) at least one fractionalization agent, and

an optional activator,
where the composition can self-disperse and produce colloidal non-layered particles when coming into contact with an aqueous fluid.

US2014/0186279 A1 discloses topical bioadhesive formulations comprising low viscosity, non-liquid crystalline, mixtures of: a) at least one neutral diacyl lipid and/or at least one tocopherol; b) at least on phospholipid; c) at least one biocompatible, oxygen containing, low viscosity organic solvent; wherein at least one bioactive agent is dissolved or dispersed in the low viscosity mixture and wherein the pre-formulation forms, or is capable of forming, at least one liquid crystalline phase structure upon contact with an aqueous fluid.

### SUMMARY OF THE INVENTION

However, in the cosmetological composition disclosed in JP2008-169219A and the composition disclosed in JP2008-509120A, the scratch resistance (the residuality on the mucous membrane when friction is applied) and the retention (attachability to the mucous membrane in a moist environment) in a case of being brought into contact with water were not at a satisfactory level as shown by Comparative Examples described later, and there was room for improvement in these compositions.

An object of the present invention is to provide a biomaterial that is excellent in scratch resistance and retention in a case of being brought into contact with water.

As a result of diligent studies to solve the above problems, the inventors of the present invention have found that the above problems can be solved by using a biomaterial including predetermined components and have completed the present invention.

That is, subject-matter of the present invention is a biomaterial as claimed in independent claim 1. Embodiments of the invention are defined in the dependent claims.

According to the present invention, it is possible to provide a biomaterial that is excellent in scratch resistance and retention in a case of being brought into contact with water.

### BRIEF DESCRIPTION OF THE DRAWINGS

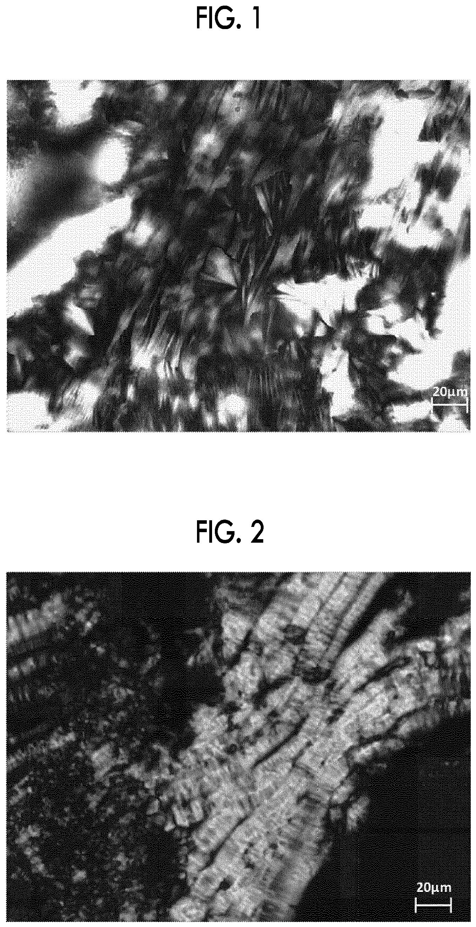

Fig. 1 is a drawing substitute photo showing a liquid crystal phase (a hexagonal columnar phase) formed of a biomaterial of Example 7 (observation magnification: 50 times).
Fig. 2 is a drawing substitute photo showing a liquid crystal phase (a lamellar phase) formed of a biomaterial of Reference Example 1 (observation magnification: 50 times).

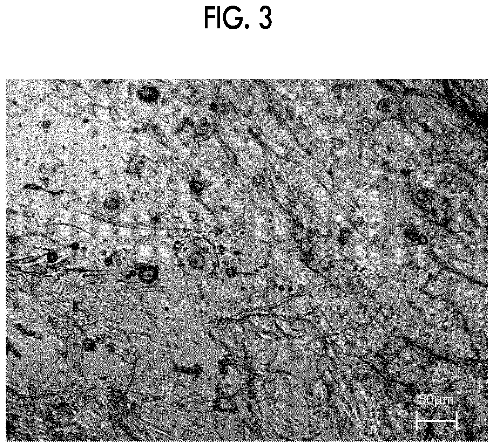

Fig. 3 is a drawing substitute photo showing a liquid crystal phase (a cubic phase) formed of a biomaterial of Reference Example 5 (observation magnification: 20 times, transmitted light).

### DESCRIPTION OF THE PREFERRED EMBODIMENTS

In the present specification, the range indicated by using "to" means a range including both ends before and after "to". For example, a range indicated by "A to B" includes A and B.

In addition, in the present specification, the solid content is intended to be a component contained in the composition excluding the dispersion medium component, and a component is calculated as a solid content even in a case where the property thereof is liquid.

### [Biomaterial]

A biomaterial according to the embodiment of the present invention includes a neutral acyl lipid, at least one selected from the group consisting of a phospholipid and a quaternary ammonium salt, and a carboxyvinyl polymer.

Hereinafter, each component of the biomaterial according to the embodiment of the present invention will be described.

### <Neutral acyl lipid>

The neutral acyl lipid means an electrically neutral acyl lipid. That is, the neutral acyl lipid does not include a cation moiety and an anion moiety. An acyl lipid means a lipid containing an acyl group.

Examples of the neutral acyl lipid include a neutral monoacyl lipid, a neutral diacyl lipid, and a neutral triacyl lipid.

In the biomaterial according to the embodiment of the present invention, the neutral acyl lipid contains both of a neutral monoacyl lipid and a neutral diacyl lipid, and a total content of the neutral monoacyl lipid and the neutral diacyl lipid is 80% by mass to 100% by mass, preferably 90% by mass to 100% by mass, and more preferably 100% by mass, with respect to the total mass of the neutral acyl lipid.

A content of the neutral monoacyl lipid is preferably 30% by mass to 70% by mass, with respect to the total mass of the neutral acyl lipid.

A content of the neutral diacyl lipid is preferably 30% by mass to 70% by mass, with respect to the total mass of the neutral acyl lipid.

A content of the neutral triacyl lipid is preferably 0% by mass to 20% by mass with respect to the total mass of the neutral acyl lipid, and it is more preferable that the neutral triacyl lipid is not contained (0% by mass).

Among them, it is more preferable that a content of the neutral monoacyl lipid is 30% by mass to 70% by mass, a content of the neutral diacyl lipid is 30% by mass to 70% by mass, with respect to the total mass of the neutral acyl lipid, and the neutral triacyl lipid is not contained.

The number of carbon atoms of the acyl group of the neutral acyl lipid is not particularly limited; however, the acyl group preferably has 6 to 32 carbon atoms and more preferably 16 to 22 carbon atoms.

The hydrocarbon group as the acyl group, excluding a carbonyl group, is preferably a saturated or unsaturated chain-type hydrocarbon group having 5 to 31 carbon atoms, and more preferably a saturated or unsaturated chain-type hydrocarbon group having 15 to 21 carbon atoms. Specific examples thereof include CH₃(CH₂)₁₄-, CH₃(CH₂)₇CH=CH(CH₂)₇-, and CH₃(CH₂)₄(CH=CHCH₂)₂(CH₂)₆-; however, the examples are not limited thereto.

In a case where a neutral acyl lipid has two or more acyl groups in one molecule, the types of acyl groups may be the same or different from each other.

Examples of the neutral acyl lipid include glycerol, diglycerol, sugar (for example, inositol), and a lipid obtained by forming an ester bond between a polyol such as succinic acid and a fatty acid. Among them, an acyl glycerol is preferable and a glyceryl oleate is more preferable.

Examples of the acyl glycerol include monoacyl glycerol, diacyl glycerol, and triacyl glycerol.

In addition, examples of the glyceryl oleate include glyceryl monooleate, glyceryl the total mass of the neutral acyl lipid.

Among them, the neutral acyl lipid preferably includes both a neutral monoacyl lipid and a neutral diacyl lipid.

A content of the neutral monoacyl lipid is not particularly limited; however, it is preferably 0% by mass to 100% by mass, more preferably 30% by mass to 70% by mass, with respect to the total mass of the neutral acyl lipid.

A content of the neutral diacyl lipid is not particularly limited; however, it is preferably 0% by mass to 100% by mass, more preferably 30% by mass to 70% by mass, with respect to the total mass of the neutral acyl lipid.

A content of the neutral triacyl lipid is not particularly limited; however it is preferably 0% by mass to 20% by mass with respect to the total mass of the neutral acyl lipid, and it is more preferable that the neutral triacyl lipid is not contained (0% by mass).

Among them, it is more preferable that a content of the neutral monoacyl lipid is 30% by mass to 70% by mass, a content of the neutral diacyl lipid is 30% by mass to 70% by mass, with respect to the total mass of the neutral acyl lipid, and the neutral triacyl lipid is not contained.

The number of carbon atoms of the acyl group of the neutral acyl lipid is not particularly limited; however, the acyl group preferably has 6 to 32 carbon atoms and more preferably 16 to 22 carbon atoms.

The hydrocarbon group as the acyl group, excluding a carbonyl group, is preferably a saturated or unsaturated chain-type hydrocarbon group having 5 to 31 carbon atoms, and more preferably a saturated or unsaturated chain-type hydrocarbon group having 15 to 21 carbon atoms. Specific examples thereof include CH₃(CH₂)₁₄-, CH₃(CH₂)₇CH=CH(CH₂)₇-, and CH₃(CH₂)₄(CH=CHCH₂)₂(CH₂)₆-; however, the examples are not limited thereto.

In a case where a neutral acyl lipid has two or more acyl groups in one molecule, the types of acyl groups may be the same or different from each other.

Examples of the neutral acyl lipid include glycerol, diglycerol, sugar (for example, inositol), and a lipid obtained by forming an ester bond between a polyol such as succinic acid and a fatty acid. Among them, an acyl glycerol is preferable and a glyceryl oleate is more preferable.

Examples of the acyl glycerol include monoacyl glycerol, diacyl glycerol, and triacyl glycerol.

In addition, examples of the glyceryl oleate include glyceryl monooleate, glyceryl dioleate, and glyceryl trioleate.

Here, the monoacyl glycerol and the glyceryl monooleate correspond to one aspect of the neutral monoacyl lipid described above, and the diacyl glycerol and the glyceryl dioleate correspond to one aspect of the neutral diacyl lipid described above, and the triacyl glycerol and the glyceryl trioleate correspond to one aspect of the neutral triacyl lipid.

In the present specification, the content proportions of the neutral monoacyl lipid, the neutral diacyl lipid, and the neutral triacyl lipid in the neutral acyl lipid are obtained from measurement with a high performance liquid chromatography (HPLC) method.
· HPLC measurement conditions
   Column: Cadenza CD-C18 (4.6 mm x 300 mm) (manufactured by Imtakt Corporation)
   Eluent: water/tetrahydrofuran
   Flow rate: 1.0 mL/min
   Detection: Corona Charged Aerosol Detector (Corona CAD)
   Column temperature: 50°C

A content of the neutral acyl lipid in the biomaterial according to the embodiment of the present invention is not particularly limited; however, it is preferably 35% by mass to 85% by mass, more preferably 35% by mass to 75% by mass, and still more preferably 55% by mass to 75% by mass, with respect to the total mass of the solid content of the biomaterial according to the embodiment of the present invention.

### <Phospholipid>

The phospholipid is not particularly limited as long as it has a phosphate ester structure in the molecular structure thereof; however, a glycerophospholipid having glycerin as a skeleton and a sphingophospholipid having sphingosine as a skeleton are typical.

In both a case of the phospholipid being a glycerophosphoric acid and a case of the phospholipid being a sphingophospholipid, an acyl group derived from a fatty acid is contained in the molecule.

The number of carbon atoms of the acyl group of the phospholipid is not particularly limited; however, the acyl group preferably has 12 to 22 carbon atoms and more preferably 16 to 18 carbon atoms.

The hydrocarbon group as the acyl group, excluding a carbonyl group, is preferably a saturated or unsaturated chain-type hydrocarbon group having 11 to 21 carbon atoms, and more preferably a saturated or unsaturated chain-type hydrocarbon group having 15 to 17 carbon atoms. Specific examples of the hydrocarbon group include CH₃(CH₂)₁₄-, CH₃(CH₂)₇CH=CH(CH₂)₇-, and CH₃(CH₂)₄(CH=CHCH₂)₂(CH₂)₆-; however, the examples are not limited thereto.

In a case where a phospholipid has two or more acyl groups in one molecule, the types of acyl groups may be the same or different from each other.

Specific examples of the phospholipid include phosphatidylcholine. The acyl group of the phosphatidylcholine is preferably an acyl group derived from palmitic acid (CH₃(CH₂)₁₄COOH), oleic acid (CH₃(CH₂)₇CH=CH(CH₂)₇COOH), or linoleic acid (CH₃(CH₂)₄(CH=CHCH₂)₂(CH₂)₆COOH). Specific examples of the phosphatidylcholine include a PO phosphatidylcholine (a phosphatidylcholine having palmitic acid at the first position (α position), oleic acid at the second position (β position), and choline at the third position (γ position)), a DL phosphatidylcholine (a phosphatidylcholine having linoleic acid at the first position (α-position), linoleic acid at the second position (β-position), and choline at the third position (γ-position)), and a dipalmitoylphosphatidylcholine.

A content of the phospholipid in the biomaterial according to the embodiment of the present invention is not particularly limited; however, the content thereof in total together with a quaternary ammonium salt described later is preferably 15% by mass to 65% by mass, more preferably 20% by mass to 60% by mass, and still more preferably 25% by mass to 45% by mass, with respect to the total mass of the solid content of the biomaterial according to the embodiment of the present invention.

### <Quaternary ammonium salt>

The quaternary ammonium salt is an ionic compound consisting of a quaternary ammonium cation and an anion.

The quaternary ammonium cation is preferably a polyatomic ion having a positive charge represented by the molecular formula NR₄⁺. Here, R's each independently represent an alkyl group or an aryl group, which may have a substituent, and a plurality of R's may be the same or different from each other.

Among them, the quaternary ammonium cation of the quaternary ammonium salt is preferably a quaternary ammonium cation represented by the following formula.

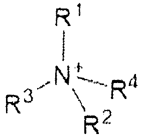

In the formula, R¹, R², R³, and R⁴ are each independently an alkyl group having 1 to 22 carbon atoms, and a hydrogen atom of the alkyl group may be substituted with an alkoxy group, an acyl group, or an acyloxy group.

The number of carbon atoms of the alkoxy group, the acyl group, or the acyloxy group, with which the hydrogen atom of the alkyl group is substituted, is not particularly limited; however, it is preferably 16 to 18.

Examples of the quaternary ammonium salt include dioleoyloxytrimethylammonium propane chloride (DOTAP, N-[1-(2,3-dioreoiloxy)propyl]-N,N,N-trimethylammonium chloride), dioctadecenyltrimethylammonium propane chloride (DOTMA, N-[1-(2,3-dioreyloxy)propyl)]-N,N,N-trimethylammonium chloride), didecyldimethylammonium chloride, didecyldimethylammonium bromide, dilauryldimethylammonium chloride, disetyldimethylammonium chloride, disetyldimethylammonium bromide, distearyldimethylammonium chloride, distearyldimethylammonium bromide, dioleyldimethylammonium chloride, dibehenyldimethylammonium chloride, dibehenyldimethylammonium bromide, dipalmitoylethyl hydroxyethylmonium metosulfate, and distearoylethyl hydroxyethylmonium metosulfate. In addition, these quaternary ammonium salts can be used as a mixture of two or more kinds thereof.

A content of the quaternary ammonium salt in the biomaterial according to the embodiment of the present invention is not particularly limited; however, the content thereof in total togther with the phospholipid described above is preferably 15% by mass to 65% by mass, more preferably 20% by mass to 60% by mass, and still more preferably 25% by mass to 45% by mass, with respect to the total mass of the solid content of the biomaterial according to the embodiment of the present invention.

### <Carboxyvinyl polymer>

In a case of being brought into contact with water, in the biomaterial according to the embodiment of the present invention, a liquid crystal phase is formed by water, a neutral acyl lipid, a phospholipid, and/or a quaternary ammonium salt.

The carboxyvinyl polymer swells when coming into contact with water, and a network of liquid crystal micelles and a network of carboxyvinyl polymers are superpositioned. In this manner, the existence of two networks of the network of liquid crystal micelles and the network of carboxyvinyl polymers can improve scratch resistance and retention in a case where the biomaterial according to the embodiment of the present invention is brought into contact with water.

The carboxyvinyl polymer is a water-soluble vinyl polymer having a carboxy group and specifically, is a polymer having a repeating unit derived from acrylic acid and/or methacrylic acid as the main chain and having a crosslinked structure of allyl sucrose or pentaerythritol (a crosslinked structure derived from the allyl sucrose or the pentaerythritol) formed by allyl ether or the like. In addition, this carboxyvinyl polymer may be a carboxyvinyl polymer salt.

Specific examples of the commercially available carboxyvinyl polymer include "CARBOPOL 941", "CARBOPOL 971", "CARBOPOL 974", "CARBOPOL 980", and "CARBOPOL 981", which are manufactured by Lubrizol Advanced Materials Inc.; "HIVISWAKO 103", "HIVISWAKO 104", and "HIVISWAKO 105", which are manufactured by FUJIFILM Wako Pure Chemical Corporation; "Junron PW-120", "Junron PW-121", and "Junron PW-312S", which are manufactured by TOAGOSEI CO., LTD.; "AQUAPEC HV-501E" and "AQUAPEC HV-505E", which are manufactured by SUMITOMO SEIKA CHEMICALS CO., LTD.; and "Synthalen K" and "Synthalen L" manufactured by 3V Sigma S.p.A.

In the biomaterial according to the embodiment of the present invention, one kind of the carboxyvinyl polymer may be used alone, or two or more kinds thereof may be used in combination.

### << Content of carboxyvinyl polymer >>

A content of the carboxyvinyl polymer in the biomaterial according to the embodiment of the present invention is not particularly limited; however, it is preferably 0.1% by mass to 10.0% by mass, more preferably 1.0% by mass to 5.0% by mass, and still more preferably 1.0% by mass to 3.5% by mass, with respect to the total mass of the solid content of the biomaterial according to the embodiment of the present invention.

### <Non-aqueous dispersion medium>

The biomaterial according to the embodiment of the present invention can further contain a non-aqueous dispersion medium.

The non-aqueous dispersion medium is not particularly limited as long as it is a non-aqueous medium capable of dispersing the at least one selected from the group consisting of a neutral acyl lipid, a phospholipid, and a quaternary ammonium salt, described above, and dispersing a carboxyvinyl polymer. The non-aqueous dispersion medium is preferably an alcohol having 7 or more carbon atoms and more preferably an alcohol having 7 to 18 carbon atoms.

Examples of the alcohol having 7 or more carbon atoms include heptyl alcohol (7 carbon atoms), capryl alcohol (8 carbon atoms), pelargonic alcohol (9 carbon atoms), capric alcohol (10 carbon atoms), lauryl alcohol (12 carbon atoms), myristyl alcohol (14 carbon atoms), oleyl alcohol (18 carbon atoms), and stearyl alcohol (18 carbon atoms).

In a case where the biomaterial according to the embodiment of the present invention includes the non-aqueous dispersion medium, the solid content in the biomaterial according to the embodiment of the present invention is not particularly limited; however, it is preferably 80% by mass or more and preferably 90% by mass or more. The upper limit is, for example, less than 100% by mass.

### <Other components>

The biomaterial according to the embodiment of the present invention may include other components as long as the effects of the present invention are not impaired, in addition to the at least one selected from the group consisting of a neutral acyl lipid, a phospholipid, and a quaternary ammonium salt, the carboxyvinyl polymer, and the non-aqueous dispersion medium, which are described above.

Examples of other components described above include sugar and sugar alcohol.

The sugar is not particularly limited; however, examples thereof include a monosaccharide and a disaccharide. Specific examples the sugar include glucose, galactose, sucrose, trehalose, and lactose.

The sugar alcohol is an organic compound having a structure in which the carbonyl group of aldose or ketose is reduced. Specific examples thereof include erythritol, xylitol, mannitol, and sorbitol, but the examples are not particularly limited.

### <Liquid crystal phase>

The biomaterial according to the embodiment of the present invention forms a liquid crystal phase when being brought into contact with water. The water is not limited to pure water, and it may be water (aqueous moisture) contained in an aqueous fluid other than water. Examples of the aqueous fluid other than water include saliva, tissue fluid, blood, and lymph, but the examples are not limited thereto.

The amount of water which is brought into contact with the biomaterial according to the embodiment of the present invention is not particularly limited; however, it is preferably 20% by mass to 1,000% by mass and more preferably 30% by mass to 500% by mass, with respect to the total mass of the biomaterial according to the embodiment of the present invention.

Here, the liquid crystal phase is not particularly limited; however, it is usually any one phase selected from the group consisting of a lamellar (La) phase, a sponge (V2) phase, a hexagonal columnar (H2) phase, a bicontinuous cubic (L3) phase, or a cubic (Q) phase, and a mixed state of two or more thereof. The liquid crystal phase is preferably a lamellar phase, a hexagonal columnar phase, or a cubic phase. The temperature at which the biomaterial according to the embodiment of the present invention is brought into contact with water is not particularly limited; however, it is preferably 20°C to 40°C and more preferably 35°C to 40°C.

### <Method for manufacturing biomaterial>

The biomaterial according to the embodiment of the present invention can be manufactured by mixing a neutral acyl lipid in which contents of a neutral monoacyl lipid and a neutral diacyl lipid are within a specific range; at least one selected from the group consisting of a phospholipid and a quaternary ammonium salt; a carboxyvinyl polymer; and, as desired, a non-aqueous dispersion medium.

The mixing method is not particularly limited, and a conventionally known mixing method can be used.

### [Use and method for using biomaterial]

The biomaterial according to the embodiment of the present invention means a material that is used in a living body, and examples thereof include a material that is used in a living body, for example, for assisting or repairing a living body an original function of which has been lost or a living body a function of which has been deteriorated due to injury or disease.

The biomaterial according to the embodiment of the present invention can be used for mucous membrane protection, particularly preferably for oral cavity mucous membrane protection.

In a case where the biomaterial according to the embodiment of the present invention is used for a mucous membrane, when the biomaterial according to the embodiment of the present invention is placed on the mucous membrane and as necessary, water or a solution containing water is added thereon, the biomaterial according to the embodiment of the present invention absorbs water to form a liquid crystal phase, thereby strongly being attached to the mucous membrane.

In particular, in a case where the biomaterial according to the embodiment of the present invention is applied to the oral cavity mucous membrane, when the biomaterial according to the embodiment of the present invention is attached to the oral cavity mucous membrane, the liquid crystal phase is formed by the aqueous moisture in saliva, and thus is handling is easy. In addition, in a case where the amount of saliva is small, water or artificial saliva may be sprayed to supply aqueous moisture after the biomaterial according to the embodiment of the present invention is attached to the oral cavity mucous membrane.

In addition, the biomaterial according to the embodiment of the present invention can be preferably used for the sustained release of a drug. For example, a pharmaceutical pre-preparation is prepared by adding a drug to the biomaterial, and the pharmaceutical pre-preparation is brought into contact with water to form a liquid crystal phase, whereby the biomaterial can be used as a drug sustainedly-releasing base material.

### Examples

Hereinafter, the present invention will be more specifically described according to Examples, but the present invention is not limited to Examples below.

### [Examples 2, 3, 4 and 6 to 14, Reference Examples 1 and 5, and Comparative Examples 1 to 8]

### <Preparation method for biomaterial>

### (Reference Example 1)

A neutral acyl lipid (1-oleoyl-rac-glycerin (> 99%), manufactured by Sigma-Aldrich Co. LLC; 5.5 g) was mixed with a phospholipid (phosphatidylcholine (Lipoid S100, manufactured by Lipoid GmbH; 3.0 g), and the mixture was heated and stirred at 60°C for 2 hours until the mixture became a yellow transparent oily liquid (hereinafter, may be referred to as a "solid content A"). After weighing a carboxyvinyl polymer (Carbopol 941, manufactured by Lubrizol Advanced Materials Inc.; 10 mg), the carboxyvinyl polymer was dispersed in a non-aqueous dispersion medium (oleyl alcohol, manufactured by Tokyo Chemical Industry Co., Ltd.; 100 mg), the required amount of the solid content A (890 mg) was added thereto, and then the resultant mixture was stirred at room temperature for 10 minutes to prepare a biomaterial.

Using the same preparation method, each component shown in Table 1 was mixed according to the blending amount shown in Table 1 to prepare biomaterials of Examples 2 to 4 and 6 to 14, Reference Example 5 and Comparative Examples 1 to 8.

Neutral acyl lipids (2), (3), (4), (6) and (7) are neutral acyl lipids according to the present invention, while neutral acyl lipids (1), (5) and (8) to (11) are not.

### <Result and evaluation>

### <<Confirmation of formation of liquid crystal phase>>

The prepared biomaterial (20 µL) was placed on a slide glass, water (about 200 µL) was sprayed thereon from above, excess water was removed therefrom, covered with a cover glass, and then an initial observation was performed using a polarizing microscope (Nikon ECLIPSE E600 POL).

Thereafter, the biomaterial was heated together with the slide glass on a hot stage (INSTEC STC200) heated in advance to 40°C, and the change in the liquid crystal phase was checked to identify the phase.

### <<Preparation of pseudo biological membrane>>

Tetradodecyl ammonium bromide (TDAB, manufactured by FUJIFILM Wako Pure Chemical Corporation; 50 mg), polyvinyl chloride (manufactured by FUJIFILM Wako Pure Chemical Corporation; 800 mg), and di-n-octylphosphonate (DOPP, manufactured by FUJIFILM Wako Pure Chemical Corporation; 0.6 mL) were dissolved in tetrahydrofuran (THF, FUJIFILM Wako Pure Chemical Corporation; 10 mL) and dried at room temperature with a petri dish to obtain a lipid membrane (about 200 µm thickness).

Next, the prepared lipid membrane was attached to a hydrogel consisting of agar (Karikorikan (registered trade mark), manufactured by Ina Food Industry Co., Ltd.; 0.5 g), gellan gum (Kelcogel (registered trade mark), manufactured by CP Kelco; 0.1 g), and distilled water (49.4 g).

Subsequently, the surface of the lipid membrane was coated with a 2-methacryloyloxyethyl phosphorylcholine (MPC) polymer (LIPIDURE (registered trade mark) - CM5206, manufactured by NOF CORPORATION) to obtain a pseudo biological membrane.

### <<Evaluation of retention>>

The prepared biomaterial was applied onto the prepared pseudo biological membrane (1 cmϕ, 500 µm thickness), artificial saliva (Saliveht (registered trade mark), manufactured by TEIJIN PHARMA LIMITED) was sprayed thereon, and the biomaterial was allowed to stand for 1 minute such that water was absorbed, thereby preparing a sample for evaluation.

The prepared sample for evaluation was placed in a petri dish and filled with artificial saliva (Saliveht) until the sample for evaluation was immersed. The petri dish was placed in a constant temperature shaker (37°C) and shaken such that the sample for evaluation was repeatedly hit to the wall of the petri dish. In this test, the time required for the biomaterial, which absorbed water from the pseudo biological membrane, to disappear by being peeled or dissolved from the pseudo biological membrane was measured, and the retention was evaluated according to the following criteria.

### (Evaluation criteria for retention)

Retained for 4 hours or more ··· A
Disappeared in 1 hour or more and less than 4 hours ··· B
Disappeared in less than 1 hour ··· C

The evaluation results are shown in the "Evaluation" column of Table 1.

### << Evaluation of scratch resistance >>

The prepared biomaterial was applied onto the prepared pseudo biological membrane (1 cmϕ, 500 µm thickness), artificial saliva (Saliveht (registered trade mark), manufactured by TEIJIN PHARMA LIMITED) was sprayed thereon, and the biomaterial was allowed to stand for 1 minute such that water was absorbed, thereby preparing a sample for evaluation.

The prepared sample for evaluation was repeatedly worn with a wear testing machine (surface property measuring machine TRIBOGEAR TYPE: 14FW, manufactured by Shinto Scientific Co., Ltd.), and the times of the reciprocation until the sample was peeled or dissolved from the pseudo biological membrane was measured, and scratch resistance was evaluated according to the following criteria. A triangular eraser core (Ain CLIC, manufactured by Pentel Co, Ltd,) was set on the head of the wear testing machine, and the test was performed under the conditions of a load of 30 g, an amplitude of 30 mm, and a speed of 6,000 mm/min.

### (Evaluation of scratch resistance)

Endured 500 times or more ··· A
Peeled after 100 times or more and less than 500 times ··· B
Peeled after less than 100 times ··· C

The evaluation results are shown in the "Evaluation" column of Table 1.

**[Table 1-1]**

| | | Mono/di/tri [% by mass] | Mono + di [% by mass] | Example (E)/Reference Example (RE) | | | | | | |
|---|---|---|---|---|---|---|---|---|---|---|
| | | | | RE1 | E2 | E3 | E4 | RE5 | E6 | E7 |
| Biomaterial | Neutral acyl lipid (1) | 100/0/0 | 100 | 58 | | | | | | |
| | Neutral acyl lipid (2) | 70/30/0 | 100 | | 58 | | | | | |
| | Neutral acyl lipid (3) | 50/50/0 | 100 | | | 58 | | | | |
| | Neutral acyl lipid (4) | 30/70/0 | 100 | | | | 58 | | | |
| | Neutral acyl lipid (5) | 0/100/0 | 100 | | | | | 58 | | |
| | Neutral acyl lipid (6) | 54/46/0 | 100 | | | | | | 58 | |
| | Neutral acyl lipid (7) | 49/42/9 | 91 | | | | | | | 58 |
| | Neutral acyl lipid (8) | 42/36/22 | 78 | | | | | | | |
| | Neutral acyl lipid (9) | 35/30/35 | 65 | | | | | | | |
| | Neutral acyl lipid (10) | 27/23/50 | 50 | | | | | | | |
| | Neutral acyl lipid (11) | 0/0/100 | 0 | | | | | | | |
| | Diglyceryl monoleate | | | | | | | | | |
| | Olive oil | | | | | | | | | |
| | Phospholipid (1) quaternary | | | 31 | 31 | 31 | 31 | 31 | 31 | 31 |
| | ammonium salt (1) | | | | | | | | | |
| | Hydrogenated lecithin | | | | | | | | | |
| | Carboxyvinyl polymer (1) | | | 1 | 1 | 1 | 1 | 1 | 1 | 1 |
| | Carboxyvinyl polymer (2) | | | | | | | | | |
| | Oleyl alcohol | | | 10 | 10 | 10 | 10 | 10 | 10 | 10 |
| | Pentylene glycol | | | | | | | | | |
| | Glycerin | | | | | | | | | |
| | Water | | | | | | | | | |
| | Total | | | 100 | 100 | 100 | 100 | 100 | 100 | 100 |
| Result | Types of liquid crystal phase (40°C) | | | La | H2 | H2 | Q | Q | H2 | H2 |
| Evaluation | Retention | | | B | A | A | A | A | A | A |
| | Scratch resistance | | | A | A | A | A | B | A | A |

| | | | | | | | | | | |
|---|---|---|---|---|---|---|---|---|---|---|
| A content of each component of a biomaterial is indicated in terms of "parts by mass". | | | | | | | | | | |

**[Table 1-2]**

| | | Mono/di/tri [% by mass] | Mono + di [% by mass] | Example | | | | | | |
|---|---|---|---|---|---|---|---|---|---|---|
| | | | | 8 | 9 | 10 | 11 | 12 | 13 | 14 |
| Biomaterial | Neutral acyl lipid (1) | 100/0/0 | 100 | | | | | | | |
| | Neutral acyl lipid (2) | 70/30/0 | 100 | | | | | | | |
| | Neutral acyl lipid (3) | 50/50/0 | 100 | | | | | | | |
| | Neutral acyl lipid (4) | 30/70/0 | 100 | | | | | | | |
| | Neutral acyl lipid (5) | 0/100/0 | 100 | | | | | | | |
| | Neutral acyl lipid (6) | 54/46/0 | 100 | | | | | | | |
| | Neutral acyl lipid (7) | 49/42/9 | 91 | 58 | 18 | 40 | 67 | 85 | 57 | 55 |
| | Neutral acyl lipid (8) | 42/36/22 | 78 | | | | | | | |
| | Neutral acyl lipid (9) | 35/30/35 | 65 | | | | | | | |
| | Neutral acyl lipid (10) | 27/23/50 | 50 | | | | | | | |
| | Neutral acyl lipid (11) | 0/0/100 | 0 | | | | | | | |
| | Diglyceryl monoleate | | | | | | | | | |
| | Olive oil | | | | | | | | | |
| | Phospholipid (1) | | | | 71 | 49 | 22 | 4 | 31 | 30 |
| | quaternary ammonium salt (1) | | | 31 | | | | | | |
| | Hydrogenated lecithin | | | | | | | | | |
| | Carboxyvinyl polymer (1) | | | 1 | 1 | 1 | 1 | 1 | 2 | 5 |
| | Carboxyvinyl polymer (2) | | | | | | | | | |
| | Oleyl alcohol | | | 10 | 10 | 10 | 10 | 10 | 10 | 10 |
| | Pentylene glycol | | | | | | | | | |
| | Glycerin | | | | | | | | | |
| | Water | | | | | | | | | |
| | Total | | | 100 | 100 | 100 | 100 | 100 | 100 | 100 |
| Result | Types of liquid crystal phase (40°C) | | | H2 | La | La | H2 | Q | H2 | H2 |
| Evaluation | Retention | | | A | B | A | A | B | A | A |
| | Scratch resistance | | | A | B | A | A | B | A | B |

| | | | | | | | | | | |
|---|---|---|---|---|---|---|---|---|---|---|
| A content of each component of a biomaterial is indicated in terms of "parts by mass". | | | | | | | | | | |

**[Table 1-3]**

| | | Mono/di/tri [% by mass] | Mono + di [% by mass] | Comparative Example | | | | | | | |
|---|---|---|---|---|---|---|---|---|---|---|---|
| | | | | 1 | 2 | 3 | 4 | 5 | 6 | 7 | 8 |
| Biomaterial | Neutral acyl lipid (1) | 100/0/0 | 100 | | | | | | | | |
| | Neutral acyl lipid (2) | 70/30/0 | 100 | | | | 58 | | | | |
| | Neutral acyl lipid (3) | 50/50/0 | 100 | | | | | 58 | | | |
| | Neutral acyl lipid (4) | 30/70/0 | 100 | | | | | | 58 | | |
| | Neutral acyl lipid (5) | 0/100/0 | 100 | | | | | | | | 70 |
| | Neutral acyl lipid (6) | 54/46/0 | 100 | | | | | | | | |
| | Neutral acyl lipid (7) | 49/42/9 | 91 | | | | | | | | |
| | Neutral acyl lipid (8) | 42/36/22 | 78 | 58 | | | | | | | |
| | Neutral acyl lipid (9) | 35/30/35 | 65 | | 58 | | | | | | |
| | Neutral acyl lipid (10) | 27/23/50 | 50 | | | 58 | | | | | |
| | Neutral acyl lipid (11) | 0/0/100 | 0 | | | | | | | 8 | |
| | Diglyceryl monoleate | | | | | | | | | | 30 |
| | Olive oil | | | | | | | | | 19 | |
| | Phospholipid (1) quaternary | | | 31 | 31 | 31 | 31 | 31 | 31 | | |
| | ammonium salt (1) | | | | | | | | | | |
| | Hydrogenated lecithin | | | | | | | | | 2 | |
| | Carboxyvinyl polymer (1) | | | 1 | 1 | 1 | | | | | |
| | Carboxyvinyl polymer (2) | | | | | | | | | 0.2 | |
| | Oleyl alcohol | | | 10 | 10 | 10 | 10 | 10 | 10 | | |
| | Pentylene glycol | | | | | | | | | 5 | |
| | Glycerin | | | | | | | | | 6 | |
| | Water | | | | | | | | | 60 | |
| | Total | | | 100 | 100 | 100 | 100 | 100 | 100 | 100.2 | 100 |
| Result | Types of liquid crystal phase (40°C) | | | H2 | | | H2 | H2 | Q | La | Q |
| Evaluation | Retention | | | B | C | C | A | A | B | C | A |
| | Scratch resistance | | | C | C | C | C | C | C | C | C |

| | | | | | | | | | | | |
|---|---|---|---|---|---|---|---|---|---|---|---|
| A content of each component of a biomaterial is indicated in terms of "parts by mass". | | | | | | | | | | | |

In Table 1, "Mono/di/tri" indicates the content ratio (in terms of % by mass) between the neutral monoacyl lipid, the neutral diacyl lipid, and the neutral triacyl lipid in the neutral acyl lipid.

In Table 1, "Mono + di" indicates the total content (in terms of % by mass) of the neutral monoacyl lipid and the neutral diacyl lipid in the neutral acyl lipid.

In Table 1, each component is as follows.
· Neutral acyl lipid (1): 1-oleoyl-rac-glycerin (> 99%, manufactured by Sigma-Aldrich Co. LLC)
· Neutral acyl lipid (5): Diolein (> 99%, manufactured by NU-CHECK-PREP, Inc.)
· Neutral acyl lipid (2): The neutral acyl lipid (1) (7.0 g) was mixed with the neutral acyl lipid (5) (3.0 g) at 40°C for 30 minutes until a homogeneous solution was obtained, thereby preparing a neutral acyl lipid (2).
· Neutral acyl lipids (3), (4), and (6): The neutral acyl lipid (1) was mixed with the neutral acyl lipid (5) according to the mass ratio shown in Table 1 in the same preparation method as that for the neutral acyl lipid (2), thereby preparing neutral acyl lipids (3), (4), and (6).
· Neutral acyl lipid (7): Monoolein (manufactured by Tokyo Chemical Industry Co., Ltd.)
· Neutral acyl lipid (11): Glyceryl trioleate (> 99%, manufactured by Sigma-Aldrich Co. LLC)
· Neutral acyl lipids (8) to (10): The neutral acyl lipid (1) and the neutral acyl lipid (5) were mixed with the neutral acyl lipid (11) according to the mass ratio shown in Table 1 in the same preparation method as that for the neutral acyl lipid (2), thereby preparing neutral acyl lipids (8) to (10).
· Diglyceryl monooleate (manufactured by RIKEN VITAMIN CO., LTD.)
· Olive oil (manufactured by FUJIFILM Wako Pure Chemical Corporation)
· Phospholipid (1): Phosphatidylcholine (Lipoid S100, manufactured by Lipoid GmbH)
· Quaternary ammonium salt (1): DOTAP [N-[1-(2,3-dioreoiloxy)propyl]-N,N,N-trimethylammonium chloride] (Lipoid DOTAP-Cl, manufactured by Lipoid GmbH)
Hydrogenated lecithin: COATSOME NC-21E (manufactured by NOF CORPORATION)
· Carboxyvinyl polymer (1): Carbopol 941 (manufactured by Lubrizol Advanced Materials Inc.)
· Carboxyvinyl polymer (2): AQUPEC N40R (manufactured by SUMITOMO SEIKA CHEMICALS CO., LTD.)
· Oleyl alcohol (manufactured by Tokyo Chemical Industry Co., Ltd.)
· Pentylene glycol
· Glycerin (manufactured by FUJIFILM Wako Pure Chemical Corporation)

In Table 1, the types of the liquid crystal phase are represented by the following abbreviations.
- La: lamellar phase
- V2: sponge phase
- H2: hexagonal columnar phase
- L3: bicontinuous cubic phase
- Q: cubic phase

### [Explanation of result]

The biomaterials of Examples 2, 3, 4 and 6 to 14 were good in retention and scratch resistance. Among them, from the comparison of Examples 2, 3, 4, 6 and Reference Examples 1, 5, it has been confirmed that the case where the neutral acyl lipid contains both of the neutral monoacyl lipid and the neutral diacyl lipid exhibits more excellent effects. In addition, from the comparison of Examples 9 to 12, it has been confirmed that the case where the total content of the phospholipid and the quaternary ammonium salt is 20% by mass to 60% by mass with respect to the total mass of the solid content of the biomaterial exhibits more excellent effects. Further, from the comparison of Examples 7, 13, and 14, it has been confirmed that the case where a content of the carboxyvinyl polymer is 1.0% by mass to 3.5% by mass exhibits more excellent effects.

On the other hand, the biomaterials of Comparative Examples 1 to 8 had insufficient scratch resistance and could not exhibit retention and scratch resistance at the same time. Among them, Comparative Examples 2, 3, and 7 were insufficient in both the retention and the scratch resistance.

It is noted that Comparative Example 7 relates to the invention disclosed in JP2008-169219A, and Comparative Example 8 relates to the invention disclosed in JP2008-509120A.

## Claims

1. A biomaterial comprising:
a neutral acyl lipid;
at least one selected from the group consisting of a phospholipid and a quaternary ammonium salt; and
a carboxyvinyl polymer,
wherein the neutral acyl lipid includes at least one selected from the group consisting of a neutral monoacyl lipid and a neutral diacyl lipid, and
a content of the at least one selected from the group consisting of the neutral monoacyl lipid and the neutral diacyl lipid is 80% by mass to 100% by mass with respect to a total mass of the neutral acyl lipid.

2. The biomaterial according to claim 1, wherein the neutral acyl lipid includes 0% by mass to 100% by mass of a neutral monoacyl lipid, 0% by mass to 100% by mass of a neutral diacyl lipid, and 0% by mass to 20% by mass of a neutral triacyl lipid, with respect to the total mass of the neutral acyl lipid.

3. The biomaterial according to claim 1 or 2, wherein an acyl group of the neutral acyl lipid has 6 to 32 carbon atoms.

4. The biomaterial according to claim 3, wherein the acyl group has 16 to 22 carbon atoms.

5. The biomaterial according to any one of claims 1 to 4, wherein the neutral acyl lipid is an acyl glycerol.

6. The biomaterial according to claim 5, wherein the acyl glycerol is a glyceryl oleate.

7. The biomaterial according to any one of claims 1 to 6, wherein an acyl group of the phospholipid has 12 to 22 carbon atoms.

8. The biomaterial according to claim 7, wherein the acyl group has 16 to 18 carbon atoms.

9. The biomaterial according to any one of claims 1 to 8, wherein a quaternary ammonium cation of the quaternary ammonium salt is represented by the following formula, 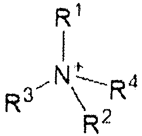 in the formula, R¹, R², R³, and R⁴ are each independently an alkyl group having 1 to 22 carbon atoms, and a hydrogen atom of the alkyl group may be substituted with an alkoxy group, an acyl group, or an acyloxy group.

10. The biomaterial according to claim 9, wherein the alkoxy group, the acyl group, or the acyloxy group, with which the hydrogen atom of the alkyl group is substituted, has 16 to 18 carbon atoms.

11. The biomaterial according to any one of claims 1 to 10, wherein the biomaterial forms a liquid crystal phase when being brought into contact with water.

12. The biomaterial according to any one of claims 1 to 11, further comprising:
a non-aqueous dispersion medium.

13. The biomaterial according to claim 12, wherein the non-aqueous dispersion medium is an alcohol having 7 or more carbon atoms.

14. The biomaterial according to any one of claims 1 to 13, wherein the biomaterial is used for mucous membrane protection.

15. The biomaterial according to claim 14, wherein the biomaterial is used for oral cavity mucous membrane protection.

16. The biomaterial according to any one of claims 1 to 15, wherein the biomaterial is used for a sustained release of a drug.

## Patentansprüche

1. Biomaterial, umfassend:
ein neutrales Acyl-Lipid;
mindestens eines, ausgewählt aus der Gruppe bestehend aus einem Phospholipid und einem quaternären Ammoniumsalz; und
ein Carboxyvinylpolymer,
wobei das neutrale Acyl-Lipid mindestens eines, ausgewählt aus der Gruppe bestehend aus einem neutralen Monoacyl-Lipid und einem neutralen Diacyl-Lipid enthält, und
ein Gehalt des mindestens einen Lipids, das ausgewählt ist aus der Gruppe bestehend aus dem neutralen Monoacyl-Lipid und dem neutralen Diacyl-Lipid, 80 Masse-% bis 100 Masse-% in Bezug auf eine Gesamtmasse des neutralen Acyl-Lipids beträgt.

2. Biomaterial nach Anspruch 1, wobei das neutrale Acyl-Lipid 0 Masse-% bis 100 Masse-% eines neutralen Monoacyl-Lipids, 0 Masse-% bis 100 Masse-% eines neutralen Diacyl-Lipids und 0 Masse-% bis 20 Masse-% eines neutralen Triacyl-Lipids, bezogen auf die Gesamtmasse des neutralen Acyl-Lipids, enthält.

3. Biomaterial nach Anspruch 1 oder 2, wobei eine Acylgruppe des neutralen Acyl-Lipids 6 bis 32 Kohlenstoffatome aufweist.

4. Biomaterial nach Anspruch 3, wobei die Acylgruppe 16 bis 22 Kohlenstoffatome aufweist.

5. Biomaterial nach einem der Ansprüche 1 bis 4, wobei das neutrale Acyl-Lipid ein Acylglycerin ist.

6. Biomaterial nach Anspruch 5, wobei das Acylglycerin ein Glyceryloleat ist.

7. Biomaterial nach einem der Ansprüche 1 bis 6, wobei eine Acylgruppe des Phospholipids 12 bis 22 Kohlenstoffatome aufweist.

8. Biomaterial nach Anspruch 7, wobei die Acylgruppe 16 bis 18 Kohlenstoffatome aufweist.

9. Biomaterial nach einem der Ansprüche 1 bis 8, wobei ein quaternäres Ammoniumkation des quaternären Ammoniumsalzes durch die folgende Formel dargestellt wird 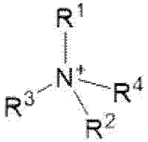 wobei in der Formel R¹, R², R³ und R⁴ jeweils unabhängig voneinander eine Alkylgruppe mit 1 bis 22 Kohlenstoffatomen sind, und ein Wasserstoffatom der Alkylgruppe substituiert sein kann mit einer Alkoxygruppe, einer Acylgruppe oder einer Acyloxygruppe.

10. Biomaterial nach Anspruch 9, wobei die Alkoxygruppe, die Acylgruppe oder die Acyloxygruppe, mit der das Wasserstoffatom der Alkylgruppe substituiert ist, 16 bis 18 Kohlenstoffatome aufweist.

11. Biomaterial nach einem der Ansprüche 1 bis 10, wobei das Biomaterial eine Flüssigkristallphase bildet, wenn es mit Wasser in Kontakt gebracht wird.

12. Biomaterial nach einem der Ansprüche 1 bis 11, ferner umfassend:
ein nicht-wässriges Dispersionsmedium.

13. Biomaterial nach Anspruch 12, wobei das nicht-wässrige Dispersionsmedium ein Alkohol mit 7 oder mehr Kohlenstoffatomen ist.

14. Biomaterial nach einem der Ansprüche 1 bis 13, wobei das Biomaterial zum Schutz einer Schleimhaut verwendet wird.

15. Biomaterial nach Anspruch 14, wobei das Biomaterial zum Schutz der Mundhöhlenschleimhaut verwendet wird.

16. Biomaterial nach einem der Ansprüche 1 bis 15, wobei das Biomaterial für eine verzögerte Freisetzung eines Arzneimittels verwendet wird.

## Revendications

1. Biomatériau, comprenant :
un lipide acyle neutre ;
au moins un élément sélectionné parmi le groupe consistant en un phospholipide et un sel d'ammonium quaternaire, et
un polymère carboxyvinylique,
dans lequel le lipide acyle neutre inclut au moins un élément sélectionné parmi le groupe consistant en un lipide monoacyle neutre et un lipide diacyle neutre, et
une teneur du au moins un élément sélectionné parmi le groupe consistant en le lipide monoacyle neutre et le lipide diacyle neutre s'étend de 80 % en masse à 100 % en masse par rapport à une masse totale du lipide acyle neutre .

2. Biomatériau selon la revendication 1, dans lequel le lipide acyle neutre inclut de 0 % en masse à 100 % en masse d'un lipide monoacyle neutre, 0 % en masse à 100 % en masse d'un lipide diacyle neutre, et 0 % en masse à 20 % en masse d'un lipide triacyle neutre, par rapport à une masse totale du lipide acyle neutre.

3. Biomatériau selon la revendication 1 ou 2, dans lequel un groupe acyle du lipide acyle neutre présente de 6 à 32 atomes de carbone.

4. Biomatériau selon la revendication 3, dans lequel le groupe acyle présente de 16 à 22 atomes de carbone.

5. Biomatériau selon l'une quelconque des revendications 1 à 4, dans lequel le lipide acyle neutre est un glycéride.

6. Biomatériau selon la revendication 5, dans lequel le glycéride est un oléate de glycéryle.

7. Biomatériau selon l'une quelconque des revendications 1 à 6, dans lequel un groupe acyle du phospholipide présente de 12 à 22 atomes de carbone.

8. Biomatériau selon la revendication 7, dans lequel le groupe acyle présente de 16 à 18 atomes de carbone.

9. Biomatériau selon l'une quelconque des revendications 1 à 8, dans lequel un cation d'ammonium quaternaire du sel d'ammonium quaternaire est représenté par la formule suivante : 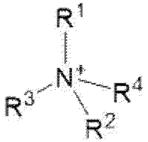 dans la formule, R¹, R², R³ et R⁴ sont chacun indépendamment un groupe alkyle présentant de 1 à 22 atomes de carbone, et un atome d'hydrogène du groupe alkyle peut être substitué par un groupe alkoxy, un groupe acyle, ou un groupe acyloxy.

10. Biomatériau selon la revendication 9, dans lequel le groupe alkoxy, le groupe acyle, ou le groupe acyloxy, par lequel l'atome d'hydrogène du groupe alkyle est remplacé, présente de 16 à 18 atomes.

11. Biomatériau selon l'une quelconque des revendications 1 à 10, dans lequel le biomatériau forme une phase de cristal liquide lorsqu'il est amené en contact avec l'eau.

12. Biomatériau selon l'une quelconque des revendications 1 à 11, comprenant en outre :
un milieu de dispersion non aqueux.

13. Biomatériau selon la revendication 12, dans lequel le milieu de dispersion non aqueux est un alcool présentant 7 atomes de carbone ou davantage.

14. Biomatériau selon l'une quelconque des revendications 1 à 13, dans lequel le biomatériau est utilisé pour une protection de muqueuse.

15. Biomatériau selon la revendication 14, dans lequel le biomatériau est utilisé pour une protection de muqueuse de cavité buccale.

16. Biomatériau selon l'une quelconque des revendications 1 à 15, dans lequel le biomatériau est utilisé pour une libération prolongée d'un médicament.
